# EUROPÄISCHE PATENTSCHRIFT

(11) **EP 0 331 092 B1**
(45) Veröffentlichungstag und Bekanntmachung des Hinweises auf die Patenterteilung: **04.08.1993**
(21) Anmeldenummer: 89103446.4
(22) Anmeldetag: 28.02.1989
(51) Int. Cl.: C07C 233/46, C07C 233/45, C07K 5/06

(54) **Neue Alpha-Aminocarbonsäure-Derivate, ihre Herstellung und Verwendung**
Alpha-amino-carboxylic-acid derivatives, their preparation and use
Dérivés d'acides alpha-amino carboxyliques, leur préparation et leur utilisation

(30) Priorität: 03.03.1988 DE 3806852
(43) Veröffentlichungstag der Anmeldung: 06.09.1989
(73) Patentinhaber: KNOLL AG, D-67061 Ludwigshafen (DE)
(72) Erfinder: Rosenberg, Joerg, Dr., D-6700 Ludwigshafen (DE); Grünhagen, Hans-Heinrich, Dr., D-6700 Ludwigshafen (DE); Lenke, Dieter, Prof. Dr., D-6700 Ludwigshafen (DE)
(74) Vertreter: Karau, Wolfgang, Dr.

(56) Entgegenhaltungen:
- EP-A- 0 162 747
- EP-A- 0 178 624
- EP-A- 0 206 947
- EP-A- 0 247 507
- CHEMICAL ABSTRACTS, Band 110, 1. Mai 1989, Seite 243, Zusammenfassung Nr. 158095j, Columbus, Ohio, US; M. GOTO et al.: "Acceleration effect of anionic surfactants on extraction rate of copper with liquid surfactant membrane containing LIX65N and nonionic surfactant"
- CHEMICAL ABSTRACTS, Band 109, 19. Dezember 1988, Seite 435, Zusammenfassung Nr. 226276a, Columbus, Ohio, US; & JP-A-62 138 502

## Beschreibung

Die vorliegende Erfindung betrifft neue α-Aminodicarbonsäure-Derivate, deren Herstellung und deren Verwendung als Wirkstoffträger.

Bei vielen Arzneimittelzubereitungen ist die Wirksamkeit insbesondere dadurch beschränkt, daß die Wirkstoffe nur geringe Halbwertszeiten im Blut besitzen und/oder einer schnellen enzymatischen Hydrolyse unterliegen. Viele Wirkstoffe zeigen auch starke Nebenwirkungen infolge unspezifischer Aufnahme derselben in verschiedene Gewebebereiche. Bei anderen Wirkstoffen - insbesondere bei Peptiden und Proteinen -, die meist nicht oral appliziert werden können, besteht der Wunsch nach einer Arzneimittelformulierung, die eine kontinuierliche Freisetzung des Wirkstoffs in die Blutbahn durch bioabbaubare Arzneimittelträger ermöglicht. Es ist bekannt, daß sich Nebenwirkungen, insbesondere bei Cancerostatica, vermeiden lassen, wenn mit Hilfe von Arzneimittelträgersystemen ein zielgerichteter Transport dieser Wirkstoffe zum Zielorgan (oder Tumor), z.B. durch Verabreichung in Form bioabbaubarer, ultrafeiner Partikel, gelingt.

Es ist weiter bekannt, daß aus den natürlich vorkommenden Phospholipiden in Wasser Vesikel (auch als Liposomen bezeichnet) gebildet werden können, die als Wirkstoffträgersystem beschrieben wurden (EP 178 624 u.a.). Solche Systeme weisen allerdings einige Nachteile auf:
- die aus natürlichem Material extrahierten Lipide stellen Lipidmischungen dar, die je nach Rohstoffquelle eine veränderliche Zusammensetzung besitzen,
- die chemische Synthese der reinen Phospholipide (Einzelsubstanzen) ist aufwendig,
- die Phospholipide sind instabil, werden leicht oxidiert bzw. durch Hydrolyse in stärker toxische Lysolecithine umgewandelt,
- die Möglichkeiten zur Bildung von Vesikeln mit besonderen Eigenschaften durch Wahl geeigneter Lipide ist wegen des begrenzten Angebots an reinen Phospholipiden begrenzt.

Von Kimura et al [Chem. Abstr. 109, 226 276a (1988] ist die Verwendung des N-(4-Oxobutansäure)-glutaminsäure-di-n-octylesters in Form seines Natriumsalzes für Agglutinationsreaktionen beschrieben worden.

Es wurde nun gefunden, daß α-Aminodicarbonsäure-Derivate der Formel I 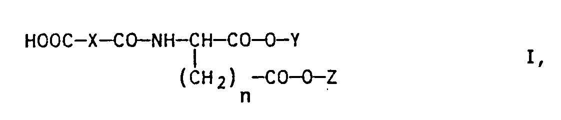
worin
- X: eine C₂-C₆-Alkylengruppe, eine -CH=CH-Gruppe oder eine gegebenenfalls acetylierte -CH₂-CHOH-, -CHOH-CHOH- oder -CH₂-CHNH₂- darstellt,
- Y und Z: aliphatische Kohlenwasserstoffreste mit 10 bis 22 C-Atomen bedeuten und
- n: die Zahl 2 oder 3 ist,
sowie deren Natrium-, Kalium- oder Ammoniumsalze besser als Wirkstoffträgersysteme geeignet sind.

Als HOOC-X-CO-Gruppe sind beispielsweise die Reste von Fumarsäure, Sebacinsäure, Malonsäure, Glutarsäure, Adipinsäure, Bernsteinsäure, Tartronsäure, Äpfelsäure, Weinsäure, Asparaginsäure oder Glutaminsäure zu nennen. Bevorzugt sind hierbei Dicarbonsäureester mit 4 Kohlenstoffatomen, die eine oder zwei acetylierte Hydroxylgruppen oder eine gegebenenfalls acetylierte Aminogruppe tragen können. Ganz besonders bevorzugt ist der Bernsteinsäurerest.

Als Aminosäurebaustein sind vor allem D-, L- oder D,L-Asparaginsäure sowie D-, L- oder D,L-Glutaminsäure geeignet.

Die Gruppen -O-Y- bzw. -O-Z leiten sich ab von geradzahligen oder ungeradzahligen, gesättigten oder ungesättigten, linearen oder verzweigten Fettalkoholen, deren Hydroxylgruppe endständig oder nicht endständig sitzt, oder die polymerisierbare Gruppen wie z.B. Dien- oder Diingruppen innerhalb der Kohlenstoffkette besitzen und die eine Länge von insgesamt 10 bis 22 Kohlenstoffatomen haben. Bevorzugt sind dabei geradzahlige oder ungeradzahlige, gesättigte oder ungesättigte Fettalkohole mit endständiger Hydroxylgruppe. Ganz besonders bevorzugt sind die Reste von 1-Dodecanol, 1-Tetradecanol, 1-Hexadecanol, 1-Octadecanol sowie 9-Octadecen-1-ol und 9,12-Octadecadien-1-ol.

Außer den Natrium- und Kaliumsalzen der neuen Verbindungen sind noch Ammoniumsalze geeignet, die sich von Ammoniak und Alkylaminen mit bis zu 6 C-Atomen in den Alkylresten ableiten.

Die erfindungsgemäßen Verbindungen lassen sich herstellen, indem man ein Aminodicarbonsäureester der Formel II 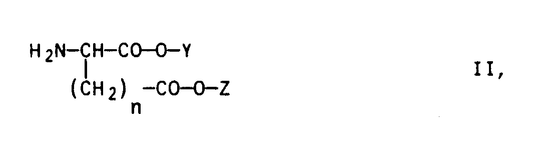
worin Y, Z und n die angegebene Bedeutung haben, mit dem Anhydrid einer Dicarbonsäure der Formel III 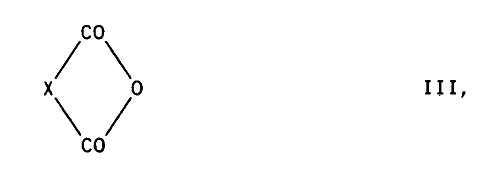
worin X die angegebene Bedeutung besitzt, umsetzt und die so erhaltenen α-Aminodicarbonsäure-Derivate gegebenenfalls in ihre Salze überführt.

Die Umsetzung wird zweckmäßig in Gegenwart einer Base, wie Pyridin, bei Raumtemperatur durchgeführt. Nach dem Ansäuern, beispielsweise mit Salzsäure, erhält man aus dem Umsetzungsprodukt die Säure I, die sich mit einer Base in ihr Salz überführen läßt.

Die Ausgangsverbindungen II sind aus der entsprechenden Aminodicarbonsäure und den Alkoholen YOH und ZOH zugänglich.

Somit lassen sich die erfindungsgemäßen Verbindungen in nur wenigen Reaktionsstufen mit einfachen, leicht durchzuführenden Synthesen in hoher Reinheit herstellen. Reine Phospholipide dagegen sind nur in mehrstufigen, aufwendigeren Reaktionen erhältlich. Zudem können die erfindungsgemäßen Verbindungen leicht enantiomerenrein, z.B. durch Einsatz der natürlich vorkommenden L-Aminosäuren, gewonnen werden (ohne Racematspaltung). Die Verwendung ausschließlich natürlich vorkommender Bausteine (Aminosäuren, Fettsäurederivate etc.) führt schließlich auch zu einer niedrigen Toxizität dieser bioabbaubaren Amphiphile. Die Kontamination mit stärker toxischen Nebenprodukten (bzw. Hydrolyseprodukten), wie bei den Phospholipiden oft (in Form von Lysolecithinen) vorhanden ist, entfällt hier ganz.

Die Verbindungen der vorliegenden Erfindung sind amphiphil, d.h. sie besitzen sowohl hydrophile als auch lipophile Gruppen. Sie aggregieren in wäßrigen Systemen oberhalb einer definierten Temperatur (in der Regel zwischen 20 und 70°C) spontan unter Ausbildung von Membrandoppelschichten, aus denen beispielsweise Vesikel einer definierten Größe bzw. Größenverteilung gebildet werden können.

Die amphiphile Struktur der erfindungsgemäßen Verbindungen ist bei den Salzen (Na, K, NH₄, NR₄) deutlich stärker ausgeprägt als bei den ungeladen vorliegenden Carbonsäuren.

Die amphiphile Natur der neuen Verbindungen erlaubt ferner die Verwendung der Substanzen zur Herstellung von Emulsionen, Mikroemulsionen, sowie von Gelen. Die aus den Verbindungen aufgebauten Membranstrukturen bestehen ganz allgemein aus Amphiphilaggregaten, in denen die polaren Kopfgruppen der Moleküle an der Grenzfläche zu einer wäßrigen Phase orientiert vorliegen. Diese Aggregate können nicht nur aus Vesikeln, sondern beispielsweise auch aus Micellen oder Mikroemulsionen bestehen.

Die Methoden zur Herstellung feiner bzw. ultrafeiner Teilchen in wäßriger Phase aus den erfindungsgemäßen Verbindungen sind im Prinzip mit denen identisch, die von vergleichbaren Amphiphilen (z.B. Phospholipiden) her bekannt sind.

Als Beispiele für die Herstellung von Vesikeln unterschiedlicher Größe seien genannt:

### Methode A

Eine abgewogene Menge an fein gepulverter erfindungsgemäßer Substanz wird in wäßriger, isotonischer und gepufferter Kochsalzlösung mit Hilfe eines Rührers zu einer trüben aber homogenen Lösung dispergiert. Diese läßt man dann langsam auf Raumtemperatur abkühlen. Im Fall von Mischungen der Verbindungen I oder beim Einbau von beispielsweise Cholesterin wird die abgewogene Mischung in wenig Methylenchlorid oder einem anderen organischen Lösungsmittel gelöst. Danach wird das Lösungsmittel im Vakuum entfernt und unter Rühren die wäßrige Phase zugegeben.

Die so erhaltene multilamellare Vesikel enthaltende Lösung kann anschließend mit Ultraschall behandelt werden, bis die gewünschte Größe der Partikel (Vesikel) erreicht ist.

Eine andere Möglichkeit, die Partikel weiter zu verkleinern, besteht darin, daß man die erhaltene Vesikellösung anschließend durch Filtermembranen definierter Porengröße preßt (Druckfiltration). Der Vorgang wird gewünschtenfalls mehrmals wiederholt, bis die Teilchen (Vesikel) die gewünschte Größe aufweisen ("Extrusionsverfahren").

### Methode B

Vesikel mit einer sehr engen Größenverteilung können z.B. auch durch kontrollierte Dialyse einer gemischten Micellenlösung aus den erfindungsgemäßen Verbindungen und geeigneten Detergentien (z.B. Octylglucose, Natriumcholat) gebildet werden, eventuell unter Zuhilfenahme der dafür auf dem Markt befindlichen Geräte (z.B. LIPOPREP®). Die Abtrennung des Detergenz aus der gemischten Micellenlösung kann allerdings auch z.B. durch Gelfiltration erfolgen.

### Methode C

Eine konzentrierte Lösung einer erfindungsgemäßen Verbindung in einem organischen Lösungsmittel wird mit Hilfe einer feinen Kanüle unter Druck in ein mit gepufferter isotonischer Kochsalzlösung gefülltes, thermostatisiertes Gefäß eingespritzt.

### Methode D

Micellenbildende, grenzflächenaktive Substanzen, z.B. ®CREMOPHOR EL (Polyethoxyethylenglyceroltriricinoleat) werden mit einer erfindungsgemäßen Verbindung gemischt. Unter Rühren wird, anfangs tropfenweise, dann in größeren Portionen, Wasser zugefügt, so daß eine klare gemischte Micellenlösung entsteht.

Die nach Methode A hergestellten Vesikel sind polydispers, die Größen liegen im Bereich von 0,1 bis 5 µm, vereinzelt sind auch größere und kleinere Vesikel vorhanden. Die so hergestellten Vesikel sind meist multilamellar und so groß, daß eine direkte Beobachtung im Lichtmikroskop möglich wird. Sie lassen sich z.B. bei der Herstellung von Depotpräparaten einsetzen, die intramuskulär verabreicht werden.

Bei der zusätzlichen Behandlung mit Ultraschall erfolgt eine Verkleinerung der Vesikelgrößen bis zu einem Grenzwert, der bei Vesikeln allgemein bei einem Durchmesser von etwa 20 Nanometer liegt. Diese "Mikrovesikel" sind unilamellar, besitzen also Membranen, die nur aus einer einzigen Bilayerschicht bestehen. Die Abnahme der Teilchengröße mit zunehmender Beschallungszeit läßt sich gut durch Laserlichtstreumessungen verfolgen.

Durch Druckfiltration polydisperser multilamellarer Vesikellösungen lassen sich durch Verwendung entsprechender Membranporengröße die Durchmesser der Vesikel vorwählen.

Die neuen Verbindungen sind bioabbaubar und besitzen, auch nach intravenöser Gabe, nur eine geringe Toxizität. Sie eignen sich daher insbesondere
- zur Herstellung langzeitstabiler wäßriger Dispersionen,
- zum Verkapseln wasserlöslicher Stoffe, insbesondere Wirkstoffen,
- als Lösungsvermittler für schwer wasserlösliche Stoffe
- zur Verbesserung der Durchdringung biologisch aktiver Stoffe durch biologische Barrieren hindurch
- zum zielgerichteten Transport von Stoffen zu bestimmten Organen, z.B. der Leber, der Lunge (auch durch Inhalation) und der Milz,
- zur Erhöhung der Selektivität und zur Verminderung der Toxizität von (Wirk)stoffen
- zur Beeinflussung der Pharmakokinetik eines Wirkstoffes durch Änderung der Freisetzung, Verteilung und Entfernung aus der systemischen Zirkulation
- zum Schutz empfindlicher (Wirk)stoffe vor chemischen Einflüssen und vor Metabolisierung sowie vor Desaktivierung
- zur Stimulierung von Immunreaktionen durch Verabreichung Vesikelverkapselter Antigene.

Für die Präparation von Vesikeln aus den erfindungsgemäßen Verbindungen sind die Salze (Na, K, NH₄, NR₄) gegenüber den Carbonsäuren bevorzugt (s. Tabelle 1). Die Carbonsäuren dagegen lassen sich gut in Mischungen mit den Salzen verwenden, um die Eigenschaften der gebildeten Vesikel zu beeinflussen (Phasenübergangstemperatur, Fluidität der Membranen, Größe der Vesikel). Inkompatibilitäten bei der Verwendung von Mischungen verschiedener Salze oder von Salzen mit verschiedenen Carbonsäuren bei der Bildung der Vesikel wurden nicht beobachtet. Die Eigenschaften der aus den erfindungsgemäßen Verbindungen hergestellten Vesikel hängen stark von den verwendeten Amphiphilen ab. So lassen sich Vesikel mit Phasenübergangstemperaturen der Membranen im Bereich 20 bis 70°C allein durch Wahl geeigneter Verbindungen (siehe Tabelle 1) leicht präparieren. Auch die Lagerstabilität derartiger Vesikellösungen (bei Raumtemperatur bzw. bei 4°C) ist von der Art bzw. der Mischung der eingesetzten Verbindung(en) abhängig und kann z.B. über ein Jahr betragen.

Bei der Anwendung der Vesikel zum Verkapseln wasserlöslicher Wirkstoffe in den wäßrigen Innenraum ist eine ausreichende Stabilität dieser Vesikel nicht nur im verwendeten Puffersystem, sondern auch in biologischen Flüssigkeiten (z.B. in Serum oder Blut bei i.v.-Injektionen) essentiell. Es ist bekannt, daß Wechselwirkungen mit Serumbestandteilen bei Phospholipidvesikeln zu einer sehr raschen Freisetzung des verkapselten Vesikelinhalts führen. Dies kann durch Einbau von Cholesterin in die Membranen verhindert werden. Es wurde nun gefunden, daß der Zusatz von Cholesterin (20 bis 70 mol%) auch bei Vesikeln aus den erfindungsgemäßen Verbindungen die zu schnelle Freisetzung des verkapselten Inhalts verhindert. Der Zusatz von Cholesterin bewirkt bei Vesikeln einiger der erfindungsgemäßen Verbindungen zusätzlich auch eine Verbesserung der Langzeit-Lagerstabilität derartiger Präparationen.

Die Stabilität der erfindungsgemäßen Präparationen ist z.B. durch Photonencorrelationsspektroskopie und Fluoreszenzanalytik nachgewiesen.

Die nachfolgenden Beispiele veranschaulichen die Erfindung.

### Beispiel 1

### Herstellung von N-(4-Oxobutansäure)-L-glutaminsäure-ditetradecylester

a) Herstellung des Ausgangsmaterials
   88,8 g (0,6 mol) L-Glutaminsäure, 256,8 g (1,2 mol) 1-Tetradecanol und 136,8 g (0,72 mol) 4-Toluolsulfonsäure-monohydrat wurden in 2500 ml Cyclohexan so lange am Wasserabscheider zum Rückfluß erhitzt, bis die berechnete Menge Wasser überdestilliert war. Nach dem Abdampfen des Lösungsmittels nahm man den Rückstand in warmem Essigsäureethylester auf und schüttelte vorsichtig mit gesättigter Natriumhydrogencarbonatlösung aus (bei Emulsionsbildung erwärmte man die Mischung oder setzte festes NaCl zum Aussalzen zu). Danach wurde die organische Phase eingedampft und der Rückstand in 2000 ml Aceton aufgenommen. Bei 30 bis 40°C wurde mit verdünnter wäßriger Salzsäure auf pH 2 eingestellt. Das ausgefallene Produkt löste man durch kurzes Aufkochen, ließ den Ansatz über Nacht stehen, saugte den Niederschlag ab, wusch diesen mit kaltem Aceton gut nach und ließ mehrere Tage bei Raumtemperatur an der Luft und anschließend im Vakuum trocknen.
   Ausbeute: 261 g (76 %)
   Schmelzpunkt: 91 bis 92°C
b) Herstellung des Endproduktes
   In einen 4 l-Kolben gab man 259 g (0,45 mol) L-Glutaminsäureditetradecylester-hydrochlorid und 1400 ml Methylenchlorid. In diese Lösung tropfte man bei Raumtemperatur 135 g Pyridin und gab unter Rühren in zwei Portionen insgesamt 54,6 g (0,546 mol) Bernsteinsäureanhydrid zu, anschließend wurde noch über Nacht weitergerührt. Nach dem Ausschütteln (je 2mal) mit 500 ml einer einmolaren Salzsäurelösung und 500 ml Wasser wurde die organische Phase über Natriumsulfat getrocknet und danach eingedampft. Der Rückstand wurde aus Methanol umkristallisiert.
   Ausbeute: 281 g (97 %)
   Schmelzpunkt: 68°C
c) Überführung in das Kaliumsalz
   256 g (0,4 mol) des Umsetzungsproduktes aus b) wurden in 1200 ml Tetrahydrofuran (THF) gelöst. Unter Rühren tropfte man bei Raumtemperatur so lange wäßrige 5 M Kaliumhydroxidlösung zu, bis der pH-Wert pH 9 bis 9,5 erreicht hatte und rührte dann noch 1 h bei Raumtemperatur nach. Dann stellte man den Kolben 1 h in Eiswasser und saugte anschließend den Niederschlag in einem Kälteraum (8°C) ab. Der abgesaugte Niederschlag wurde noch mit eiskaltem THF gewaschen und an der Luft getrocknet.
   Ausbeute: 223 g (78 %)
   Schmelzpunkt: 165 bis 167°C

### Beispiel 2

### Herstellung von N-(4-Oxobutansäure)-L-asparginsäuredioleylester

a) Herstellung des Ausgangsmaterials
   23,6 g (0,088 mol) Oleylalkohol, 5,3 g (0,04 mol) Asparginsäure und 8,4 g (0,044 mol) p-Toluolsulfonsäure-Hydrat wurden in 120 ml Cyclohexan 17 h am Wasserabscheider unter Stickstoff und unter Rückfluß erhitzt. Das Lösungsmittel wurde dann im Vakuum entfernt und der Rückstand in Essigsäureethylester aufgenommen. Die organische Phase wurde nach dem Ausschütteln mit gesättigter Natriumhydrogencarbonatlösung über Natriumsulfat getrocknet. Schließlich wurde HCl-Gas eingeleitet, bis ein pH-Wert von etwa 2 erreicht war (Eiskühlung). Das ausgefallene Produkt wurde kalt abgesaugt, aus Aceton umkristallisiert und im Vakuum getrocknet.
   Ausbeute: 17,2 g (64 %)
   Schmelzpunkt: 53 bis 55°C
b) Herstellung des Endprodukts
   16,1 g (0,024 mol) L-Asparaginsäuredioleylester-Hydrochlorid wurden in 80 ml Methylenchlorid und 21 ml Pyridin gelöst. Dazu gab man unter Rühren portionsweise 2,9 g (0,029 mol) Bernsteinsäureanhydrid und rührte 5 h bei Raumtemperatur nach. Nach dem Ausschütteln (2mal mit 100 ml 1 M HCl, 3mal mit Wasser) wurde die organische Phase über Natriumsulfat getrocknet und eingedampft. Den öligen Rückstand löste man in 150 ml Acetonitril und ließ unter heftigem Rühren und Eiskühlung kristallisieren. Der Niederschlag wurde nach 45 min kalt abgesaugt, aus Acetonitril kristallisiert und im Vakuum getrocknet.
   Ausbeute: 15,6 g (89 %)
   Schmelzpunkt: 42 bis 43°C
c) Überführung in das Kaliumsalz
   7,4 g (0,01 mol) des Umsetzungsproduktes aus b) wurden in 30 ml Tetrahydrofuran gelöst. Unter pH-Kontrolle wurde unter Rühren so lange 5 N Kalilauge zugetropft, bis ein pH-Wert von 9,3 erreicht war, dann wurde noch 1 h nachgerührt. Man gab tropfenweise unter starkem Rühren langsam Acetonitril zu (ca. 30 ml insgesamt). Schließlich wurde auf -20 bis -30°C abgekühlt und 30 min bei dieser Temperatur nachgerührt. Den ausgefallenen Niederschlag saugte man in der Kälte vorsichtig ab, wusch mit kaltem Acetonitril und trocknete den wachsartigen Rückstand im Vakuum.
   Ausbeute: 7,1 g (91 %) Wachs

Analog Beispiel 1 und 2 wurden aus den entsprechenden L-Aminosäuren folgende Verbindungen der Formel I hergestellt:

| Beispiel Nr. | X | n | Y | Z | Salz/Säure | F (°C) |
|---|---|---|---|---|---|---|
| 2a | 2 | 2 | C₁₄ | C₁₄ | Na-Salz | 150-154 |
| 2b | 2 | 2 | C₁₄ | C₁₄ | NH₄-Salz | 92- 94 |
| 3a | 3 | 2 | C₁₄ | C₁₄ | Säure | 63 |
| 3b | 3 | 2 | C₁₄ | C₁₄ | K-Salz | 180 |
| 4a | 2 | 2 | C₁₆ | C₁₆ | Säure | 74- 75 |
| 4b | 2 | 2 | C₁₆ | C₁₆ | K-Salz | 150-153 |
| 5a | 2 | 2 | C₁₈ | C₁₈ | Säure | 82- 83 |
| 5b | 2 | 2 | C₁₈ | C₁₈ | K-Salz | 155-157 |
| 5c | 2 | 2 | C₁₈ | C₁₈ | NH₄-Salz | 85- 87 |
| 6 | 2 | 1 | C₁₀ | C₁₀ | Säure | 70- 72 |
| 7a | 2 | 1 | C₁₄ | C₁₄ | Säure | 71- 72 |
| 7b | 2 | 1 | C₁₄ | C₁₄ | K-Salz | 160-163 |
| 8a | 3 | 1 | C₁₄ | C₁₄ | Säure | 68 |
| 8b | 3 | 1 | C₁₄ | C₁₄ | K-Salz | 174-175 |
| 9a | 2 | 1 | C₁₆ | C₁₆ | Säure | 76- 77 |
| 9b | 2 | 1 | C₁₆ | C₁₆ | K-Salz | 155-157 |
| 10 | 3 | 1 | C₁₈ | C₁₈ | Säure | 80- 81 |
| 11a | 2 | 1 | C₁₈ | C₁₈ | Säure | 82- 84 |
| 11b | 2 | 1 | C₁₈ | C₁₈ | K-Salz | 145-147 |
| 12a | 2 | 1 | C₁₈ | C₁₈ | Säure | 42- 43 |
| 12b | 2 | 1 | C₁₈ | C₁₈ | K-Salz | (Wachs) |
| 12c | 2 | 1 | C₁₈ | C₁₈ | NH₄-Salz | 42- 43 |
| 13 | 2 | 2 | C₁₈ | C₁₈ | Säure | ≦ 25 (Öl) |
| 13a | 2 | 2 | C₁₈ | C₁₈ | K-Salz | (Wachs) |
| 13b | 2 | 2 | C₁₈ | C₁₈ | Na-Salz | (Wachs) |
| 14a | 2 | 1 | C₂₂ | C₂₂ | Säure | 92 |
| 14b | 2 | 1 | C₂₂ | C₂₂ | K-Salz | 122-126 |

In der Tabelle ist für Y und Z die Zahl der C-Atome angegeben. Die Reste Y und Z sind geradkettig und mit Ausnahme der Beispiele 12 und 13 gesättigt. Y und Z in Beispiel 12 und 13 sind CH₃-(CH₂)₇-CH=CH-(CH₂)₇-.

### Anwendungsbeispiel

### Verkapselung eines wasserlöslichen Farbstoffes

100 mg Substanz des Beispiels 7b (K-Salz) wurden in 20 ml Diisopropylether gelöst. Nach Zugabe von 1,0 ml einer 25 mM 6-Carboxyfluoresceinlösung (Natriumsalz, in Pufferlösung: 0,9 % NaCl + 10 mM Phosphat, pH 7,2) emulgierte man durch Beschallung. Am Rotationsverdampfer entfernte man dann vorsichtig das organische Lösungsmittel und dispergierte den Rückstand mit 10 ml der oben genannten Pufferlösung durch Schwenken im Wasserbad bei 55°C zu einer trüben aber homogenen Lösung. Nach dem Abkühlen gab man 1,0 ml auf eine Gelfiltrationssäule (®Sephadex G 50 coarse, Φ 1,5 cm, Länge 11 cm, Elution mit Pufferlösung). Die zuerst eluierte Fraktion enthielt die farbstoffhaltigen Vesikel. Zu allen Fraktionen gab man 100 µl einer 10 %igen wäßrigen ®Triton-x-100 Lösung, erwärmte kurz auf 50-60°C und bestimmt dann die Extinktion bei 492 nm, um den Anteil des verkapselten Farbstoffes zu bestimmen (in % der ursprünglich zugesetzten Menge). Beim hier beschriebenen Fall lag dieser Wert bei 34 ± 2 %. Setzte man den Farbstoff erst nach der Vesikelbildung zu (Emulsion in Pufferlösung, Farbstoffzusatz vor der Gelfiltration), erhielt man eine farblose Vesikelfraktion.

## Patentansprüche (Patentansprüche für folgende(n) Vertragsstaat(en): AT, BE, CH, DE, FR, GB, IT, LI, NL)

1. α-Aminodicarbonsäure-Derivate der Formel I 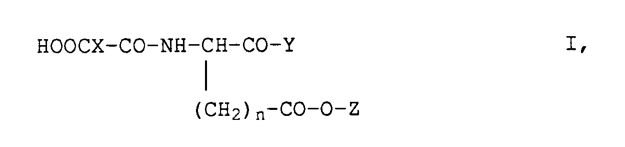 worin
X eine C₂-C₆-Alkylengruppe, eine -CH=CH-Gruppe oder eine gegebenenfalls acetylierte -CH₂-CHOH-, -CHOH-CHOH- oder -CH₂-CHNH₂- darstellt,
Y und Z aliphatische Kohlenwasserstoffreste mit 10 bis 22 C-Atomen bedeuten und
n eine Zahl 1 oder 2 ist,
sowie deren Natrium, Kalium- oder Ammoniumsalze.

2. Verfahren zur Herstellung der α-Aminodicarbonsäure-Derivate der Formel I gemäß Anspruch 1, dadurch gekennzeichnet, daß man ein Aminodicarbonsäureester der Formel II 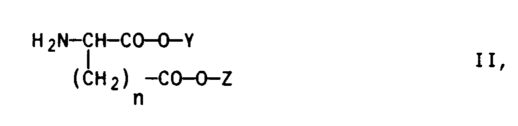 worin Y, Z und n die angegebene Bedeutung haben, mit dem Anhydrid einer Dicarbonsäure der Formel III 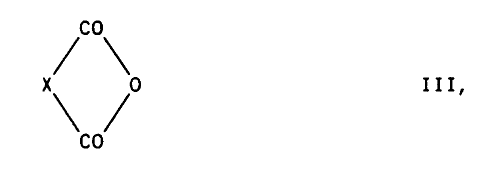 worin X die angegebene Bedeutung hat, umsetzt und die so erhaltenen α-Aminodicarbonsäure-Derivate gegebenenfalls in ihre Salze überführt.

3. Verwendung der α-Aminodicarbonsäure-Derivate der Formel I gemäß Anspruch 1 als Trägermaterial.

## Patentansprüche (Patentansprüche für folgende(n) Vertragsstaat(en): ES)

1. Verfahren zur Herstellung der α-Aminodicarbonsäure-Derivate der Formel I 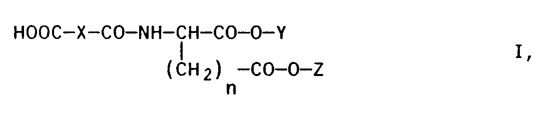 worin
X eine C₂-C₆-Alkylengruppe, eine -CH=CH-Gruppe oder eine gegebenenfalls acetylierte -CH₂-CHOH-, -CHOH-CHOH- oder -CH₂-CHNH₂- Gruppe darstellt,
Y und Z aliphatische Kohlenwasserstoffreste mit 10 bis 22 C-Atomen bedeuten und
n die Zahl 1 oder 2 ist,
sowie deren Natrium-, Kalium- oder Ammoniumsalze, dadurch gekennzeichnet, daß man einen Aminodicarbonsäureester der Formel II 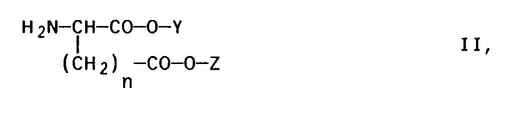 worin Y, Z und n die angegebene Bedeutung haben, mit dem Anhydrid einer Dicarbonsäure der Formel III 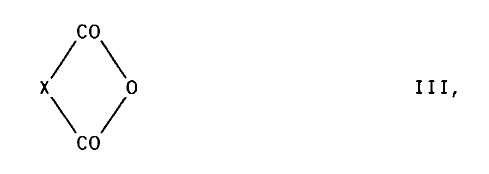 worin X die angegebene Bedeutung hat, umsetzt und die so erhaltenen α-Aminodicarbonsäure-Derivate gegebenenfalls in ihre Salze überführt.

## Claims (Claims for the following Contracting State(s): AT, BE, CH, DE, FR, GB, IT, LI, NL)

1. An α-aminodicarboxylic acid derivative of the formula I 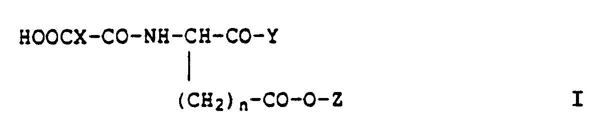 where X is C₂-C₆-alkylene, -CH=CH- or unacetylated or acetylated -CH₂-CHOH-, -CHOH-CHOH- or -CH₂-CHNH₂-, Y and Z are each an aliphatic hydrocarbon radical of 10 to 22 carbon atoms and n is 1 or 2, and its sodium, potassium or ammonium salts.

2. A process for the preparation of an α-aminodicarboxylic acid derivative of the formula I as claimed in claim 1, wherein an aminodicarboxylic ester of the formula II 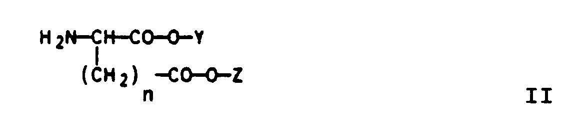 where Y, Z and n have the stated meanings, is reacted with an anhydride of a dicarboxylic acid of the formula III 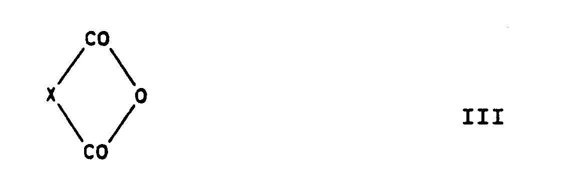 where X has the stated meanings, and, if required, the resulting α-aminodicarboxylic acid derivative is converted into its salts.

3. Use of an α-aminodicarboxylic acid derivative of the formula I as claimed in claim 1 as a carrier.

## Claims (Claims for the following Contracting State(s): ES)

1. A process for the preparation of an α-aminodicarboxylic acid derivative of the formula I 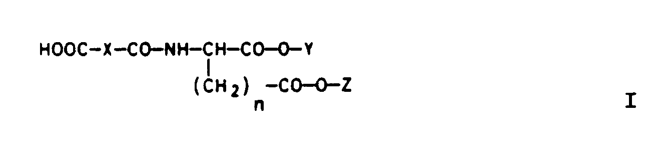 where X is C₂-C₆-alkylene, -CH=CH- Or unacetylated or acetylated -CH₂-CHOH-, -CHOH-CHOH- or -CH₂-CHNH₂-, Y and Z are each an aliphatic hydrocarbon radical of 10 to 22 carbon atoms and n is 1 or 2, and its sodium, potassium or ammonium salts, wherein an aminodicarboxylic ester of the formula II 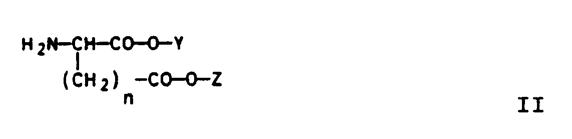 where Y, Z and n have the stated meanings, is reacted with an anhydride of a dicarboxylic acid of the formula III 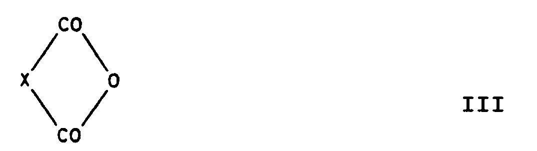 where X has the stated meanings, and, if required, the resulting α-aminodicarboxylic acid derivative is converted into its salts.

## Revendications (Revendications pour l'(les) Etat(s) contractant(s) suivant(s): AT, BE, CH, DE, FR, GB, IT, LI, NL)

1. Dérivés d'acides α-aminodicarboxyliques de formule I 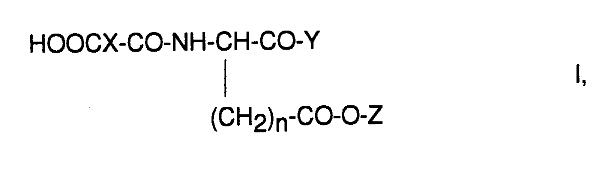 dans laquelle
X représente un groupe alkylène en C2-C6, un groupe -CH=CH- ou un groupe - CH₂-CHOH-, -CHOH-CHOH- ou -CH₂-CHNH₂- éventuellement acétylè,
Y et Z représentent des radicaux hydrocarbonés aliphatiques en C10-C22 et n est égal à 1 ou 2,
et leurs sels de sodium, de potassium ou d'ammonium.

2. Procédé de préparation des dérivés d'acides α-aminodicarboxyliques de formule I de la revendication 1, caractérisé en ce que l'on fait réagir un ester d'acide aminodicarboxylique de formule II 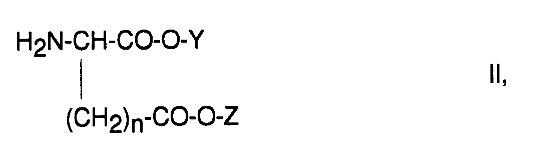 dans laquelle Y, Z et n ont les significations indiquées ci-dessus, avec l'anhydride d'un acide dicarboxylique de formule III 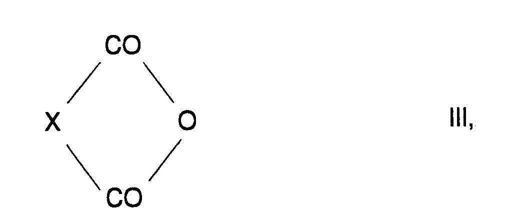 dans laquelle X a les significations indiquées ci-dessus, et le cas échéant on convertit les dérivés d'acides α-aminodi-carboxyliques ainsi obtenus en leurs sels.

3. Utilisation des dérivés d'acides α-aminodi-carboxyliques de formule I de la revendication 1 en tant que véhicules.

## Revendications (Revendications pour l'(les) Etat(s) contractant(s) suivant(s): ES)

1. Procédé de préparation des dérivés d'acides α-aminodicarboxyliques de formule I 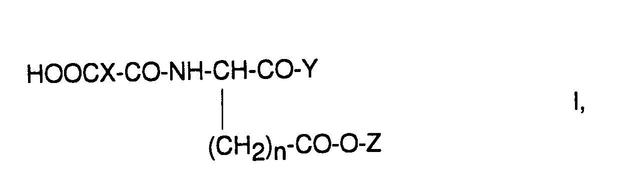 dans laquelle
X représente un groupe alkylène en C2-C6, un groupe -CH=CH- ou un groupe - CH₂-CHOH-, -CHOH-CHOH- ou -CH₂-CHNH₂- éventuellement acétylè,
Y et Z représentent des radicaux hydrocarbonés aliphatiques en C10-C22 et n est égal à 1 ou 2,
et de leurs sels de sodium, de potassium ou d'ammonium, caractérisé en ce que l'on fait réagir un ester d'acide aminodicarboxylique de formule II. 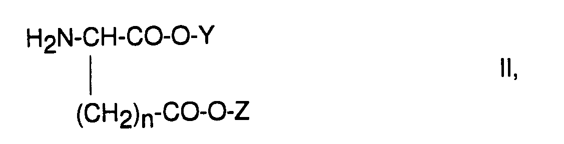 dans laquelle Y, Z et n ont les significations indiquées ci-dessus, avec l'anhydride d'un acide dicarboxylique de formule III 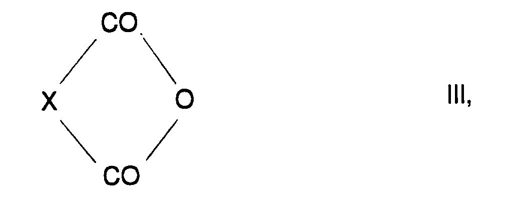 dans laquelle X a les significations indiquées ci-dessus, et le cas échéant on convertit les dérivés d'acides α-aminodi-carboxyliques ainsi obtenus en leurs sels.
